# EUROPEAN PATENT SPECIFICATION

(11) **EP 0 624 578 B1**
(45) Date of publication and mention of the grant of the patent: **23.12.1998**
(21) Application number: 94300801.1
(22) Date of filing: 03.02.1994
(51) Int. Cl.: C07D 301/32, C07D 303/04

(54) **Purification of propylene oxide**
Reinigung von Propylenoxid
Purification de l'oxyde de propylène

(30) Priority: 19.04.1993 US 47910
(43) Date of publication of application: 17.11.1994
(73) Proprietor: Huntsman Specialty Chemicals Corporation, Austin, Texas 78761 (US)
(72) Inventor: Meyer, Robert Allen, St. Louis, Mobile 63123 (US); Mueller, Mark Allen, Austin, Texas 78732 (US); Smith, William Alan, Houston, Texas 77068 (US); DeMoll, Gregory Brian, LaGrange Park, Illinois 60525 (US)
(74) Representative: Goddar, Heinz J., Dr.

(56) References cited:
- US-A- 3 881 996
- US-A- 5 133 839
- US-A- 5 262 017

## Description

The invention relates to the multi-stage purification of propylene oxide. More particularly, this invention relates to a multi-stage distillation process for removing contaminating quantities of impurities from propylene oxide. Still more particularly, this invention relates to a multi-stage distillation process for the purification of impure propylene oxide contaminated with impurities including water and other oxygen-containing impurities, e.g. propylene, propane, acetaldehyde, methyl formate, propionaldehyde, hexenes, acetone, hexanes, methanol, t-butyl alcohol, pentane, isopentane, pentenes, isopropyl alcohol and t-butyl formate. The impure propylene oxide is purified by a multi-stage extractive distillation process using a C₂ to C₆ alkylene glycol and a C₂ to C₈ alkane hydrocarbon as extractive distillation agents in separate stages.

It is known to use alkane hydrocarbons having 6 to 18 carbon atoms as extractive distillation agents in the purification of propylene oxide, (see, for example, US-A-3338800, US-A-3464897, US-A-3607669 and US-A-3843488). US-A-3464897 shows that an alkane such as octane is effective for the removal of hydrocarbon impurities having 6 carbon atoms, such as 2-methyl pentane, 4-methyl pent-1-ene, 2-methyl pent-1-ene and 2-methyl pent-2-ene. US-A-3843488 shows that alkanes having 8 to 20 carbon atoms, preferably 8 to 10 carbon atoms, such as n-octane, are effective for removing hydrocarbon impurities having 5 to 7 carbon atoms from propylene oxide.

It is also known that alkylene glycols having 2 to 6 carbon atoms can be used as extractive distillation agents in the purification of propylene oxide. Thus, US-A-3578568 discloses the use of ethylene glycol and propylene glycol; US-A-3838020 discloses the use of butylene glycols; US-A-5000825 discloses the use of glycols having 2 to 4 carbon atoms, such as ethylene glycol, propane diol or butane diol; US-A-5139622 discloses the use of triethylene glycol; and US-A-5160587 discloses the use of dipropylene glycol.

The use of multi-stage distillation processes for the purification of propylene oxide has also been proposed.

US-A-3881996 discloses a multi-stage process for the purification of propylene oxide, including a first fractionation wherein light impurities such as acetaldehyde are removed overhead, followed by a second distillation step, wherein heavy impurities such as propionaldehyde are removed in order to provide a second distillate fraction, which is then extractively distilled in the presence of octane zone to provide pure propylene oxide, and to remove alkane hydrocarbon impurities such as C₆ carbon atom impurities from the propylene oxide. In that process it is important to use the proper sequence of distillation steps since, for example, removal of heavy impurities, such as propionaldehyde, before the removal of light impurities, such as acetaldehyde, will lead to adverse results.

US-A-5133839 discloses a multi-stage process for the purification of propylene oxide utilizing a conventional distillation zone which preferably contains two distillation columns, as in US-A-3881996, followed by extractive distillation of the resulting partially-purified propylene oxide in two sequential extractive distillation columns, using either isoctane or a lower alkylene glycol as the extractive distillation agent, and removing lighter impurities in the first of the extractive distillation columns, and heavier impurities from the second of the extractive distillation columns.

US-A-4971661 discloses a multi-stage process for the purification of propylene oxide, but different extractive distillation agents, such as water and acetone, are used.

As illustrated by the prior art just discussed, it is already known to use multi-stage distillation for the purification of propylene oxide, and to use lower alkylene glycols and higher alkanes as extractive distillation agents. Thus, it was known that the use of hydrocarbons having 8 to 10 carbon atoms as extractive distillation agents was particularly effective for removing hydrocarbon contaminants such as hydrocarbons having 5 to 7 carbon atoms, from propylene oxide. It is also known to use lower alkylene glycols, which are polar solvents, for the extractive distillation in order to remove oxygenated impurities.

Pentanes and pentenes are present as minor impurities in crude propylene oxide. It has been discovered in accordance with the present invention that the removal of pentanes and pentenes presents a difficult problem.

Thus, as is pointed out, for example in US-A-3881996, the sequence of distillation steps employed in the plural stage purification of propylene oxide is crucial for effective purification. Reversing or otherwise altering the sequence of steps can lead to adverse results.

Pentanes, isopentane and pentenes may be removed like other hydrocarbon impurities by using octane in an extractive distillation. The octane lowers the relative volatility of the hydrocarbons compared to propylene oxide. The degree of relative volatility change is proportional to the amount of octane solvent present. Using typical solvent to feed ratios as disclosed in the prior art and in this Application, it has been discovered that pentanes and pentenes are not easily removed and tend to remain in the propylene oxide (PO) product. The present Application discloses that a more economical and efficient means of removing pentanes and pentenes is by a conventional distillation upstream of the octane extractive distillation. The C5 stripper tower should not be located downstream of the octane extractive distillation, since the final PO product would then be a bottoms product from the stripper. It is advantageous to take high purity products off the top of columns, rather than from the bottom.

Thus, when partially purified propylene oxide containing contaminating quantities of hydrocarbons having 5 to 7 carbon atoms (such as pentanes, pentenes, hexanes, hexenes, heptanes and heptenes) is subjected to extractive distillation using an alkane hydrocarbon extractive distillation agent, such as octane, a significant portion of the pentanes and pentenes will remain with the overhead purified propylene oxide, rather than being separated therefrom for withdrawal from the bottom of the tower, together with the extractive distillation agent and the other impurities. As a consequence, a subsequent distillation step would normally be required for the removal of the pentanes and pentenes.

It has been discovered in accordance with the present invention, however, that the use of a different sequence of distillation steps from that disclosed in the prior art will obviate this problem, so that there is no need for an additional distillation tower downstream of the alkane hydrocarbon extractive distillation.

The present invention provides a distillation process for the purification of impure propylene oxide contaminated with water, hydrocarbons and oxygen-containing impurities, which comprises the steps of:
charging said impure propylene oxide feedstock to a first extractive distillation column,
charging a C₂ to C₆ alkylene glycol first extractive distillation agent to said first extractive distillation column at a feed point above the point of introduction of said impure propylene oxide,
fractionating said impure propylene oxide in said first extractive distillation column to provide a first lighter distillation fraction, containing all of the propylene oxide and impurities boiling with and above propylene oxide, and a first heavier distillation fraction, containing water and oxygen-containing impurities and all of the extractive distillation agent,
fractionating said first lighter distillation fraction in a second distillation column, to provide a second lighter distillation fraction, containing impurities boiling above propylene oxide, and a second heavier distillation fraction, containing substantially all of the propylene oxide, and heavier boiling impurities,
charging said second heavier distillation fraction to a third extractive distillation column, and charging a C₇ to C₁₀ alkane hydrocarbon extractive distillation agent to said third extractive distillation column at a feed point above the point of introduction of said second heavier distillation fraction,
fractionating said second heavier distillation fraction in said third extraction distillation column, to provide a third lighter distillation fraction, containing impurities boiling above propylene oxide, and a third heavier distillation fraction containing substantially all of the propylene oxide, extractive distillation agent, and heavier hydrocarbon impurities,
charging said third heavier distillation fraction to a fourth extractive distillation column, and charging a C₇ to C₁₀ alkane hydrocarbon extractive distillation agent to said fourth extractive distillation column at a feed point above the point of introduction of said third heavier distillation fraction, and
fractionating said third heavier distillation fraction in said fourth extraction distillation column to provide a fourth lighter distillation fraction consisting essentially of propylene oxide, and a fourth heavier distillation fraction containing substantially all of the extractive distillation agent.

In a preferred embodiment, the first heavier distillation fraction is charged to a fifth distillation column, wherein it is fractionated to provide a fifth lighter distillation fraction containing water, and oxygen-containing impurities, and a fifth heavier distillation fraction, containing substantially all of the alkylene glycol extractive distillation agent, which is recycled to said first distillation column as said first extractive distillation agent.

Even more preferably, the third lighter distillation fraction and said fourth heavier distillation fraction are charged to a sixth distillation column and fractionated to provide a sixth lighter distillation fraction, containing lighter impurities, and a sixth heavier distillation fraction, containing substantially all of the C₇ to C₈ alkane extractive distillation agent, said sixth heavier distillation fraction being recycled to said third and fourth distillation columns as said hydrocarbon extractive distillation agents.

According to preferred embodiments of the invention:
the pressure and temperature at the top of the first distillation column (10) are 69 to 207 kPa (10 to 30 psia) and 32 to 54°C (90 to 130°F), and the pressure and temperature at the bottom of the first distillation column are 138 to 345 kPa (20 to 50 psia) and 149 to 193°C (300 to 380°F);
the pressure and temperature at the top of the second distillation column (20) are 241 to 310 kPa (35 to 45 psia) and 43 to 54°C (110 to 130°F), and the pressure and temperature at the bottom of the second distillation column are 276 to 345 kPa (40 to 50 psia) and 66 to 71°C (150 to 160°F);
the pressure and temperature at the top of the third distillation column (30) are 138 to 276 kPa (20 to 40 psia) and 54 to 71°C (130 to 160°F), and the pressure and temperature at the bottom of the third distillation column are 207 to 345 kPa (30 to 50 psia) and 82 to 104°C (180 to 220°F);
the pressure and temperature at the top of the fourth distillation column (40) are 69 to 207 kPa (10 to 30 psia) and 38 to 54°C (100 to 130°F), and the pressure and temperature at the bottom of the fourth distillation column are 172 to 310 kPa (25 to 45 psia) and 121 to 143°C (250 to 290°F);
the pressure and temperature at the top of the fifth distillation column (50) are 0.69 to 6.9 kPa (0.1 to 1.0 psia) and 32 to 54°C (90 to 130°F), and the pressure and temperature at the bottom of the fifth distillation column are 0.69 to 7.6 kPa (0.1 to 1.1 psia) and 149 to 182°C (300 to 360°F); and

The pressure and temperature at the top of the sixth distillation column (60) are 103 to 207 kPa (15 to 30 psia) and 38 to 60°C (100 to 140°F), and the pressure and temperature at the bottom of the sixth distillation column are 138 to 276 kPa (20 to 40 psia) and 93 to 149°C (200 to 300°F).

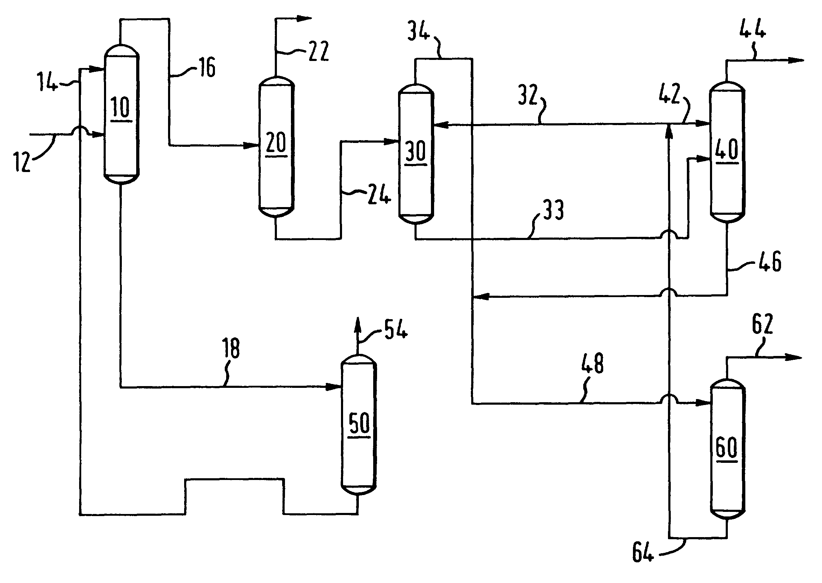

The accompanying Drawing is a schematic flow sheet, with conventional parts omitted, showing the general recovery sequence that is used in accordance with a preferred embodiment of the present invention to purify propylene oxide.

In the Drawing, conventional parts, such as valves, pumps, temperature sensors, pressure sensors, heaters, coolers, flow control regulation apparatus, reboilers, and reflux condensers, have been omitted.

The impure propylene oxide to be purified in accordance with the present invention is typically propylene oxide prepared by the reaction of tertiary butyl hydroperoxide with propylene in the presence of a molybdenum catalyst to provide a reaction mixture comprising unreacted propylene, unreacted tertiary butyl hydroperoxide, tertiary butyl alcohol, propylene oxide, and impurities. This reaction product is separated in a distillation zone (not shown) into a plurality of fractions including a propylene recycle fraction, an impure propylene oxide fraction, a tertiary butyl alcohol fraction, and a residue fraction.

The impure propylene oxide obtained in this fashion is suitably used as a feedstock for the present invention, and will normally be contaminated with one or more impurities from the group including water, propylene, propane, acetaldehyde, methyl formate, propionaldehyde, hexenes, acetone, hexanes, methanol, tertiary butyl alcohol, pentane, isopentane (i.e., crude ash methyl butane), pentenes, isopropyl alcohol and tertiary butyl formate.

The impure propylene oxide will normally contain from 97 to 99 wt.% of propylene oxide, the balance being impurities such as those enumerated above. It will be understood that some of the enumerated impurities need not always be present in impure propylene oxide, and that other impurities, not listed, may be present in minor quantities. The impurities, broadly speaking, comprise water, hydrocarbons such as propylene, propane, hexenes, hexanes, pentane, isopentane and pentenes, and oxygenated impurities including aldehydes, alcohols and esters. Both the hydrocarbon and the oxygenated impurities will normally have 2 to 6 carbon atoms.

In accordance with the present invention, an impure propylene oxide of the type described is charged to a first extractive distillation column 10 by way of a charge line 12. The fractional distillation column 10 may suitably comprise from 40 to 80 theoretical trays, and the charge line 12 for the impure propylene oxide will normally enter the column from 10 to 30 trays from the bottom.

An alkylene glycol extractive distillation agent is also charged to the column 10 by a line 14. The alkylene glycol extractive distillation agent may suitably be an alkylene glycol containing from 2 to 6 carbon atoms such as ethylene glycol, propylene glycol, 1,4-butane diol, 2-methyl-1,3-propane diol, diethylene glycol, triethylene glycol or dipropylene glycol. Preferred extractive distillation agents include triethylene glycol and dipropylene glycol.

The alkylene glycol extractive agent 14 will suitably be charged to the extractive distillation column 10 in an amount of 2 to 7 parts of impure propylene oxide per part of alkylene glycol.

Extractive distillation conditions are adjusted in the extractive distillation tower 10 so as to provide for the recovery overhead, or as a distillate fraction, of substantially all of the propylene oxide charged to the extractive distillation column 10. For example, the impure propylene oxide, which will suitably comprise 98.5 wt.% propylene oxide, the balance being impurities as mentioned above, may be charged at a temperature of 38 to 71°C (100 to 160°F).

The temperature at the bottom of the extractive distillation column 10 may suitably be 149 to 193°C (300 to 380°F) and the pressure may be 138 to 345 kPa (20 to 50 psia). The temperature at the top of the extractive distillation column 10 may, for example, be 32 to 54°C (90 to 130°F) and the pressure may suitably be 69 to 207 kPa (10 to 30 psia). A first lighter distillation fraction comprising impure propylene oxide is removed from the column 10 by way of a line 16, and may suitably comprise more than 99 wt.% propylene oxide, the balance being impurities such as acetaldehyde, methyl formate, hexenes, hexanes, methanol, water, pentanes, isopentanes and pentenes. A heavier distillation fraction 18, discharged at the bottom of the extractive distillation column 10, will comprise such impurities as water, propionaldehyde, acetone, methanol, tertiary butyl alcohol, isopropyl alcohol, tertiary butyl formate, and the alkylene glycol extractive distillation agent.

The first lighter distillation fraction 16 is charged to a second distillation column 20, which may suitably contain from 20 to 40 theoretical trays, preferably at a location 8 to 16 theoretical trays from the bottom of the column. Distillation conditions are adjusted in column 20 to provide a second lighter distillation fraction, discharged by way of a line 22, and a second heavier distillation fraction, discharged by a line 24. Distillation conditions are suitably adjusted in the distillation column 20 so that substantially all of the propylene oxide will be present in the second heavier distillation fraction 24. Typically, the lighter distillation fraction 22 will comprise impurities, such as propylene, propane, acetaldehyde and, significantly, substantially all of the pentanes and pentenes charged to the distillation column 20. The second heavier distillation fraction will typically contain substantially all of the propylene oxide, hexenes and hexanes, and only residual quantities of pentanes and pentenes.

Preferably the temperature and pressure at the top of the second distillation column are 241 to 310 kPa (35 to 45 psia) and 43 to 54°C (110 to 130°F), and the pressure and temperature at the bottom of the second distillation column are 276 to 345 kPa (40 to 50 psia) and 66 to 71°C (150 to 160°F).

The second heavier distillation fraction 24 is charged to a third distillation column 30, and a C₇ to C₈ alkane hydrocarbon extractive distillation agent is charged by a line 32.

The alkane extractive distillation agent may suitably be charged to the third distillation column 30 in an amount of 5 to 7 parts of extractive distillation agent per part of second heavier distillation fraction 24. Extractive distillation conditions are adjusted in the distillation column 30, to provide for a fourth heavier distillation fraction 33, containing substantially all of the propylene oxide, hexenes, hexanes, and residual pentenes and pentanes, and a lighter distillation fraction 34, which will typically comprise impurities such as methyl formate, acetaldehyde, water and methanol. Residual quantities of propylene oxide and hexane may also be present.

The extractive distillation agent charged to the distillation column 30 is suitably an alkane having 8 carbon atoms, such as normal octane or isooctane. A minor amount of an alkane such as nonane (usually less than 1 wt.%) may also be present in the extractive distillation agent. The second heavier distillation fraction 24 may be suitably charged to the third distillation column 30 at a temperature of 54 to 82°C (130 to 180°F). The temperature at the bottom of the extractive distillation column 30 may suitably be 82 to 104°C (180 to 320°F) and the pressure may suitably be 207 to 345 kPa (30 to 50 psia). The temperature adjacent the top of the extractive distillation column 30 may suitably be 54 to 71°C (130 to 160°F) and the pressure may suitably be 138 to 276 kPa (20 to 40 psia).

The third heavier distillation fraction 33 is charged to a fourth extractive distillation tower 40, together with an alkane extractive distillation agent, such as octane, which is charged by way of line 42. The extractive distillation agent 42 may be charged to the extractive distillation column 40 in an amount of 0.4 to 0.8 parts per part of third heavier distillation fraction 33.

Distillation conditions are adjusted in the distillation column 40 to provide a lighter distillation fraction 44, consisting essentially of propylene oxide, and a heavier distillation fraction 46, comprising the hexenes, hexanes, residual pentenes, residual pentanes and the extractive distillation agent.

Suitably, the temperature at the top of the distillation column 40 may be 38 to 54°C (100 to 130°F) and the pressure may be 69 to 207 kPa (10 to 30 psia). The temperature adjacent the bottom of the distillation column 40 may suitably be 121 to 143°C (250 to 290°F) and the pressure may suitably be 172 to 310 kPa (25 to 45 psia).

The first heavier distillation fraction 18 from the first distillation column 10 may be charged to a fifth distillation column 50 containing from 20 to 60 theoretical trays, where it may be separated under distillation conditions adjusted to provide for the recovery of substantially all of the alkylene glycol extractive distillation agent, as a heavier distillation fraction discharged through line 14, and a lighter distillation fraction 54 suitably containing substantially all of the water, acetone, tertiary butyl alcohol, methanol and isopropylene alcohol contained in the first heavier fraction 18.

The pressure at the top of the fifth distillation column 50 may suitably be 0.69 to 6.9 kPa (0.1 to 1.0 psia) and the temperature may suitably be 32 to 54°C (90 to 130°F). The pressure at the bottom of the distillation column 50 may suitably be 0.69 to 7.6 kPa (0.1 to 1.1 psia) and the temperature may suitably be 149 to 182°C (300 to 360°F).

The third lighter distillation fraction 34 and the fourth heavier distillation fraction 46 may suitably be charged by way of a line 48 to a sixth distillation column 60, which may comprise, for example, from 30 to 50 theoretical trays, at a location at least 20 theoretical trays from the bottom of the column. Distillation conditions within the distillation column 60 are adjusted to provide for the recovery of a sixth lighter distillation fraction 62 comprising remaining impurities, e.g. acetaldehyde, methyl formate, hexenes, methanol and a minor amount of octane (and possibly small amounts of residual propylene oxide), and a sixth heavier distillation fraction 64 comprising substantially all the extractive distillation agent charged to the distillation column 60 by the line 48. For example, the distillation conditions established in the sixth distillation column 60 may include a pressure at the top of the column of 103 to 207 kPa (15 to 30 psia) and a temperature of 38 to 60°C (100 to 140°F), preferably 43 to 60°C (110 to 140°F), and a pressure at the bottom of the column of 138 to 276 kPa (20 to 40 psia) and a temperature of 93 to 149°C (200 to 300°F).

### EXAMPLES

The invention will be further illustrated by the following specific Example. Where parts are mentioned, they are parts by weight.

1000 parts of an impure propylene oxide feedstock 12 containing about 98.5 wt.% of propylene oxide, and contaminated with impurities including water, propylene, propane, acetaldehyde, methyl formate, propionaldehyde, hexenes, acetone, hexanes, methanol, t-butyl alcohol, n-pentane, isopentane (i.e., 2-methyl butane), pentene, isopropyl alcohol and tertiary butyl formate and separated under extractive distillation conditions in column 10 in the presence of a triethylene glycol extractive distillation agent, into a first lighter distillation fraction 16 (about 986 parts) containing more than 99 wt.% of propylene oxide and less than 1 wt.% of contaminants including propylene, propane acetaldehyde, methyl formate, hexenes, hexanes, pentenes, pentanes, water and methanol, and a first heavier distillation fraction 18 containing water, propionaldehyde, acetone, tertiary butyl alcohol, tertiary butyl formate, isopropyl alcohol, methanol and the alkylene glycol and the triethylene glycol extractive distillation agent. The first heavier distillation fraction 18 is separated in fifth distillation column 50 into a heavier fraction 14 comprising the triethylene glycol extractive distillation agent, which is recycled to the extractive distillation column 10, and a lighter overhead fraction 54 comprising water, acetone, tertiary butyl alcohol, methanol and isopropyl alcohol.

The lighter distillation fraction 16 is separated in the second distillation column 20 into about 985 parts of a heavier distillation fraction 24 and about 1 part of a lighter distillation fraction 22 containing propylene, propane, acetaldehyde, pentenes and pentanes.

A second heavier distillation fraction 24 discharged from the distillation column 20 will comprise substantially all of the propylene oxide, hexenes, hexanes, and only residual quantities of pentanes and pentenes. This fraction is charged by way of the line 24 to a third distillation column 30 together with about 5620 parts of octane charged to the distillation column 30 by a line 32. The distillation column 30 is operated in the manner described above to provide a lighter distillation fraction 34 (about 1 part) and a heavier distillation fraction 33 (about 6604 parts). The fraction 34 will comprise acetaldehyde, methyl formate, methanol, water and residual quantities of propylene oxide and hexane. The third heavier distillation fraction 33 will comprise propylene oxide and substantially all of the octane charged to the third distillation column 30.

The third heavier distillation fraction 33 is charged to the fourth extractive distillation column 40, where it is separated together with about 3690 parts of octane in the manner described above, into about 985 parts of an overhead distillation fraction consisting essentially of propylene oxide, and containing very low quantities of the impurities present in the feedstock charged by the line 12 to the first distillation column 10. A heavier distillation fraction 46 discharged from the column 40 will comprise about 9309 parts of octane, hexenes, hexanes and residual quantities of propylene oxide.

## Claims

1. A distillation process for the purification of impure propylene oxide (12) contaminated with water, hydrocarbons and oxygen-containing impurities characterised by the steps of:
charging said impure propylene oxide feedstock (12) to a first extractive distillation column (10),
charging a C₂ to C₆ alkylene glycol first extractive distillation agent (14) to said first extractive distillation column (10) at a feed point above the point of introduction of said impure propylene oxide (12),
fractionating said impure propylene oxide in said first extractive distillation column (10) to provide a first lighter distillation fraction (16), containing all of the propylene oxide and impurities boiling with and above propylene oxide, and a first heavier distillation fraction (18), containing water and oxygen-containing impurities and all of the extractive distillation agent,
fractionating said first lighter distillation fraction (16) in a second distillation column (20), to provide a second lighter distillation fraction (22), containing impurities boiling above propylene oxide, and a second heavier distillation fraction (24), containing substantially all of the propylene oxide, and heavier boiling impurities,
charging said second heavier distillation fraction (24) to a third extractive distillation column (30), and charging a C₇ to C₁₀ alkane hydrocarbon extractive distillation agent (32) to said third extractive distillation column (30) at a feed point above the point of introduction of said second heavier distillation fraction,
fractionating said second heavier distillation fraction in said third extraction distillation column (30), to provide a third lighter distillation fraction (34), containing impurities boiling above propylene oxide, and a third heavier distillation fraction (33) containing substantially all of the propylene oxide, extractive distillation agent, and heavier hydrocarbon impurities,
charging said third heavier distillation fraction (33) to a fourth extractive distillation column (40), and charging a C₇ to C₁₀ alkane hydrocarbon extractive distillation agent (42) to said fourth extractive distillation column (40) at a feed point above the point of introduction of said third heavier distillation fraction, and
fractionating said third heavier distillation fraction in said fourth extraction distillation column (40) to provide a fourth lighter distillation fraction (44) consisting essentially of propylene oxide, and a fourth heavier distillation fraction (46) containing substantially all of the extractive distillation agent.

2. A method according to Claim 1 characterised in that said first heavier distillation fraction (18) is charged to a fifth distillation column (50), wherein it is fractionated to provide a fifth lighter distillation fraction (54) containing water, and oxygen-containing impurities, and a fifth heavier distillation fraction (14), containing substantially all of the alkylene glycol extractive distillation agent, which is recycled to said first distillation column (10) as said first extractive distillation agent.

3. A method according to Claim 1 or 2 characterised in that said third lighter distillation fraction (34) and said fourth heavier distillation fraction (46) are charged to a sixth distillation column (60) and fractionated to provide a sixth lighter distillation fraction (62), containing lighter impurities, and a sixth heavier distillation fraction (64), containing substantially all of the C₇ to C₈ alkane extractive distillation agent, said sixth heavier distillation fraction (64) being recycled to said third (30) and fourth (40) distillation columns as said hydrocarbon extractive distillation agents (32, 42).

4. A method according to any one of Claims 1 to 3 characterised in that the pressure and temperature at the top of the first distillation column (10) are 69 to 207 kPa (10 to 30 psia) and 32 to 54°C (90 to 130°F), and the pressure and temperature at the bottom of the first distillation column are 138 to 345 kPa (20 to 50 psia) and 149 to 193°C (300 to 380°F).

5. A method according to any one of Claims 1 to 4 characterised in that the pressure and temperature at the top of the second distillation column (20) are 241 to 310 kPa (35 to 45 psia) and 43 to 54°C (110 to 130°F), and the pressure and temperature at the bottom of the second distillation column are 276 to 345 kPa (40 to 50 psia) and 66 to 71°C (150 to 160°F).

6. A method according to any one of Claims 1 to 5 characterised in that the pressure and temperature at the top of the third distillation column (30) are 138 to 276 kPa (20 to 40 psia) and 54 to 71°C (130 to 160°F), and the pressure and temperature at the bottom of the third distillation column are 207 to 345 kPa (30 to 50 psia) and 82 to 104°C (180 to 220°F).

7. A method according to any one of Claims 1 to 6 characterised in that the pressure and temperature at the top of the fourth distillation column (40) are 69 to 207 kPa (10 to 30 psia) and 38 to 54°C (100 to 130°F), and the pressure and temperature at the bottom of the fourth distillation column are 172 to 310 kPa (25 to 45 psia) and 121 to 143°C (250 to 290°F).

8. A method according to Claim 2 characterised in that the pressure and temperature at the top of the fifth distillation column (50) are 0.69 to 6.9 kPa (0.1 to 1.0 psia) and 32 to 54°C (90 to 130°F), and the pressure and temperature at the bottom of the fifth distillation column are 0.69 to 7.6 kPa (0.1 to 1.1 psia) and 149 to 182°C (300 to 360°F).

9. A method according to Claim 3 characterised in that the pressure and temperature at the top of the sixth distillation column (60) are 103 to 207 kPa (15 to 30 psia) and 38 to 60°C (100 to 140°F), and the pressure and temperature at the bottom of the sixth distillation column are 138 to 276 kPa (20 to 40 psia) and 93 to 149°C (200 to 300°F).

## Patentansprüche

1. Ein Destillationsverfahren zur Reinigung von unreinem Propylenoxid (12), das mit Wasser, Kohlenwasserstoffen und sauerstoffhaltigen Verunreinigungen verunreinigt ist, gekennzeichnet durch die Schritte:
Zuführen besagten unreinen Propylenoxid-Beschickungsmaterials (12) zu einer ersten Extraktivdestillationskolonne (10),
Zuführen eines ersten Extraktivdestillationsmittels aus C₂ bis C₆-Alkylenglykol (14) zu besagter ersten Extraktivdestillationskolonne (10) an einem Zuführpunkt oberhalb des Einführungspunktes besagten unreinen Propylenoxids (12),
Fraktionieren besagten unreinen Propylenoxids in besagter ersten Extraktivdestillationskolonne (10), um eine erste leichtere Destillationsfraktion (16), die das gesamte Propylenoxid und Verunreinigungen, die mit oder oberhalb von Propylenoxid sieden, enthält, und eine erste schwerere Destillationsfraktion (18), die Wasser und sauerstoffhaltige Verunreinigungen und das gesamte Extraktivdestillationsmittel enthält, zu liefern,
Fraktionieren besagter ersten leichteren Destillationsfraktion (16) in einer zweiten Destillationskolonne (20), um eine zweite leichtere Destillationsfraktion (22), die Verunreinigungen enthält, die oberhalb von Propylenoxid sieden, und eine zweite schwerere Destillationsfraktion (24), die im wesentlichen das gesamte Propylenoxid und höhersiedende Verunreinigungen enthält, zu liefern,
Zuführen besagter zweiten schwereren Destillationsfraktion (24) zu einer dritten Extraktivdestillationskolonne (30) und Zuführen eines C₇ bis C₁₀-Alkankohlenwasserstoff-Extraktivdestillationsmittels (32) zu besagter dritten Extraktivdestillationskolonne (30) an einem Zuführpunkt oberhalb des Einführungspunktes besagter zweiten schwereren Destillationsfraktion,
Fraktionieren besagter zweiten schwereren Destillationsfraktion in besagter-dritten Extraktionsdestillationskolonne (30), um eine dritte leichtere Destillationsfraktion (34), die Verunreinigungen enthält, die oberhalb von Propylenoxid sieden, und eine dritte schwerere Destillationsfraktion (33), die im wesentlichen das gesamte Propylenoxid, Extraktivdestillationsmittel und schwerere Kohlenwasserstoff-Verunreinigungen enthält, zu liefern,
Zuführen besagter dritten schwereren Destillationsfraktion (33) zu einer vierten Extraktivdestillationskolonne (40) und Zuführen eines C₇ bis C₁₀-Alkankohlenwasserstoff-Extraktivdestillationsmittels (42) zu besagter vierten Extraktivdestillationskolonne (40) an einem Zuführpunkt oberhalb des Einführungspunktes besagter dritten schwereren Destillationsfraktion und
Fraktionieren besagter dritten schwereren Destillationsfraktion in besagter vierten Extraktionsdestillationskolonne (40), um eine vierte leichtere Destillationsfraktion (44), die im wesentlichen aus Propylenoxid besteht, und eine vierte schwerere Destillationsfraktion (46), die im wesentlichen das gesamte Extraktivdestillationsmittel enthält, zu liefern.

2. Ein Verfahren nach Anspruch 1, dadurch gekennzeichnet, daß besagte erste schwerere Destillationsfraktion (18) einer fünften Destillationskolonne (50) zugeführt wird, in der sie fraktioniert wird, um eine fünfte leichtere Destillationsfraktion (54), die Wasser und sauerstoffhaltige Verunreinigungen enthält, und eine fünfte schwerere Destillationsfraktion (14), die im wesentlichen das gesamte Alkylenglykol-Extraktivdestillationsmittel enthält, das zu besagter ersten Destillationskolonne (10) als besagtes erstes Extraktivdestillationsmittel rückgeführt wird, zu liefern.

3. Ein Verfahren nach Anspruch 1 oder 2, dadurch gekennzeichnet, daß besagte dritte leichtere Destillationsfraktion (34) und besagte vierte schwerere Destillationsfraktion (46) einer sechsten Destillationskolonne (60) zugeführt und fraktioniert werden, um eine sechste leichtere Destillationsfraktion (62), die leichtere Verunreinigungen enthält, und eine sechste schwerere Destillationsfraktion (64), die im wesentlichen das gesamte C7 bis C₈-Alkan-Extraktivdestillationsmittel enthält, zu liefern, wobei besagte sechste schwerere Destillationsfraktion (64) zu besagter dritten (30) und vierten (40) Destillationskolonne als besagte Kohlenwasserstoff-Extraktivdestillationsmittel (32, 42) rückgeführt wird.

4. Ein Verfahren nach einem der Ansprüche 1 bis 3, dadurch gekennzeichnet, daß der Druck und die Temperatur im Kopfraum der ersten Destillationskolonne (10) 69 bis 207 kPa (10 bis 30 psia) und 32 bis 54°C (90 bis 130°F) betragen und der Druck und die Temperatur im Bodenraum der ersten Destillationskolonne 138 bis 345 kPa (20 bis 50 psia) und 149 bis 193°C (300 bis 380°F) betragen.

5. Ein Verfahren nach einem der Ansprüche 1 bis 4, dadurch gekennzeichnet, daß der Druck und die Temperatur im Kopfraum der zweiten Destillationskolonne (20) 241 bis 310 kPa (35 bis 45 psia) und 43 bis 54°C (110 bis 130°F) betragen und der Druck und die Temperatur im Bodenraum der zweiten Destillationskolonne 276 bis 345 kPa (40 bis 50 psia) und 66 bis 71°C (150 bis 160°F) betragen.

6. Ein Verfahren nach einem der Ansprüche 1 bis 5, dadurch gekennzeichnet, daß der Druck und die Temperatur im Kopfraum der dritten Destillationskolonne (30) 138 bis 276 kPa (20 bis 40 psia) und 54 bis 71°C (130 bis 160°F) betragen und der Druck und die Temperatur im Bodenraum der dritten Destillationskolonne 207 bis 345 kPa (30 bis 50 psia) und 82 bis 104°C (180 bis 220°F) betragen.

7. Ein Verfahren nach einem der Ansprüche 1 bis 6, dadurch gekennzeichnet, daß der Druck und die Temperatur im Kopfraum der vierten Destillationskolonne (40) 69 bis 207 kPa (10 bis 30 psia) und 38 bis 54°C (100 bis 130°F) betragen und der Druck und die Temperatur im Bodenraum der vierten Destillationskolonne 172 bis 310 kPa (25 bis 45 psia) und 121 bis 143°C (250 bis 290°F) betragen.

8. Ein Verfahren nach Anspruch 2, dadurch gekennzeichnet, daß der Druck und die Temperatur im Kopfraum der fünften Destillationskolonne (50) 0,69 bis 6,9 kPa (0,1 bis 1,0 psia) und 32 bis 54°C (90 bis 130°F) betragen und der Druck und die Temperatur im Bodenraum der fünften Destillationskolonne 0,69 bis 7,6 kPa (0,1 bis 1,1 psia) und 149 bis 182°C (300 bis 360°F) betragen.

9. Ein Verfahren nach Anspruch 3, dadurch gekennzeichnet, daß der Druck und die Temperatur im Kopfraum der sechsten Destillationskolonne (60) 103 bis 207 kPa (15 bis 30 psia) und 38 bis 60°C (100 bis 140°F) betragen und der Druck und die Temperatur im Bodenraum der sechsten Destillationskolonne 138 bis 276 kPa (20 bis 40 psia) und 93 bis 149°C (200 bis 300°F) betragen.

## Revendications

1. Procédé de distillation pour la purification d'oxyde de propylène (12) impur contaminé par de l'eau, des hydrocarbures et des impuretés contenant de l'oxygène caractérisé par les étapes de :
chargement de ladite alimentation en oxyde de propylène impur (12) vers une première colonne d'extraction par distillation (10),
chargement d'un premier alkylène glycol en C₂ à C₆ comme agent d'extraction par distillation (14) vers ladite première colonne d'extraction par distillation (10) en un point d'alimentation supérieur au point d'introduction dudit oxyde de propylène impur (12),
fractionnement dudit oxyde de propylène impur dans ladite première colonne d'extraction par distillation (10) pour donner une première fraction légère de distillation (16), contenant tout l'oxyde de propylène et les impuretés à ébullition égale et supérieure à l'oxyde de propylène, et une première fraction lourde de distillation (18), contenant l'eau et les impuretés contenant de l'oxygène et tout l'agent d'extraction par distillation,
fractionnement de ladite première fraction légère de distillation (16) dans une seconde colonne de distillation (20), pour donner une seconde fraction légère de distillation (22), contenant les impuretés à ébullition supérieure à l'oxyde de propylène, et une seconde fraction lourde de distillation (24), contenant sensiblement tout l'oxyde de propylène, et les impuretés à ébullition plus lourde,
chargement de ladite seconde fraction lourde de distillation (24) vers une troisième colonne d'extraction par distillation (30), et chargement d'un hydrocarbure alcane en C₇ à C₁₀ agent d'extraction par distillation (32) dans ladite troisième colonne d'extraction par distillation (30) en un point d'alimentation supérieur au point d'introduction de ladite seconde fraction lourde de distillation,
fractionnement de ladite seconde fraction lourde de distillation dans ladite troisième colonne d'extraction par distillation (30), pour donner une troisième fraction légère de distillation (34), contenant les impuretés à ébullition supérieure à l'oxyde de propylène, et une troisième fraction lourde de distillation (33) contenant sensiblement tout l'oxyde de propylène, l'agent d'extraction par distillation, et les impuretés hydrocarbonées plus lourdes,
chargement de ladite troisième fraction lourde de distillation (33) dans une quatrième colonne d'extraction par distillation (40), et chargement d'un hydrocarbure alcane en C₇ à C₁₀ comme agent d'extraction par distillation (42) dans ladite quatrième colonne d'extraction par distillation (40) en un point d'alimentation supérieur au point d'introduction de ladite troisième fraction lourde de distillation, et
fractionnement de ladite troisième fraction lourde de distillation dans ladite quatrième colonne d'extraction par distillation (40) pour donner une quatrième fraction légère de distillation (44) constituée essentiellement d'oxyde de propylène, et une quatrième fraction lourde de distillation (46) contenant sensiblement tout l'agent d'extraction par distillation.

2. Procédé selon la revendication 1 caractérisé en ce que ladite première fraction lourde de distillation (18) est chargée vers une cinquième colonne de distillation (50), dans laquelle elle est fractionnée pour donner une cinquième fraction légère de distillation (54) contenant de l'eau, et des impuretés contenant de l'oxygène, et une cinquième fraction lourde de distillation (14), contenant sensiblement tout l'alkylène glycol agent d'extraction par distillation, qui est recyclé vers ladite première colonne de distillation (10) comme étant ledit premier agent d'extraction par distillation.

3. Procédé selon la revendication 1 ou 2 caractérisé en ce que ladite troisième fraction légère de distillation (34) et ladte quatrième fraction lourde de distillation (46) sont chargées vers une sixième colonne de distillation (60) et fractionnées pour donner une sixième fraction légère de distillation (62), contenant les impuretés légères, et une sixième fraction lourde de distillation (64), contenant sensiblement tout l'alcane en C7 à C8 agent d'extraction par distillation, ladite sixième fraction lourde de distillation (64) étant recyclée vers lesdites troisième (30) et quatrième (40) colonnes de distillation comme étant lesdits hydrocarbures agents d'extraction par distillation (32, 42).

4. Procédé selon l'une quelconque des revendications 1 à 3 caractérisé en ce que la pression et la température en tête de la première colonne de distillation (10) sont de 69 à 207 kPa (10 à 30 psia) et de 32 à 54°C (90 à 130°F), et la pression et la température en bas de la première colonne de distillation sont de 138 à 345 kPa (20 à 50 psia) et de 149 à 193°C (300 à 380°F).

5. Procédé selon l'une quelconque des revendications 1 à 4 caractérisé en ce que la pression et la température en tête de la seconde colonne de distillation (20) sont de 241 à 310 kPa (35 à 45 psia) et de 43 à 54°C (110 à 130°F), et la pression et la température en bas de la seconde colonne de distillation sont de 276 à 345 kPa (40 à 50 psia) et de 66 à 71°C (150 à 160°F).

6. Procédé selon l'une quelconque des revendications 1 à 5 caractérisé en ce que la pression et la température en tête de la troisième colonne de distillation (30) sont de 138 à 276 kPa (20 à 40 psia) et de 54 à 71°C (130 à 160°F), et la pression et la température en bas de la troisième colonne de distillation sont de 207 à 345 kPa (30 à 50 psia) et de 82 à 104°C (180 à 220°F).

7. Procédé selon l'une quelconque des revendications 1 à 6 caractérisé en ce que la pression et la température en tête de la quatrième colonne de distillation (40) sont de 69 à 207 kPa (10 à 30 psia) et de 38 à 54°C (100 à 130°F), et la pression et la température en bas de la quatrième colonne de distillation sont de 172 à 310 kPa (25 à 45 psia) et de 121 à 143°C (250 à 290°F).

8. Procédé selon la revendication 2 caractérisé en ce que la pression et la température en tête de la cinquième colonne de distillation (50) sont de 0,69 à 6,9 kPa (0,1 à 10 psia) et de 32 à 54°C (90 à 130°F), et la pression et la température en bas de la cinquième colonne de distillation sont de 0,69 à 7,6 kPa (0,1 à 1,1 psia) et de 149 à 182°C (300 à 360°F).

9. Procédé selon la revendication 3 caractérisé en ce que la pression et la température en tête de la sixième colonne de distillation (60) sont de 103 à 207 kPa (15 à 30 psia) et de 38 à 60°C (100 à 140°F), et la pression et la température en bas de la sixième colonne de distillation sont de 138 à 276 kPa (20 à 40 psia) et de 93 à 149°C (200 à 300°F).
